# EUROPEAN PATENT SPECIFICATION

(11) **EP 4 642 444 B1**
(45) Date of publication and mention of the grant of the patent: **11.03.2026**
(21) Application number: 24813427.2
(22) Date of filing: 27.11.2024
(51) Int. Cl.: A61K 31/045, A61P 15/00, A61P 15/10

(54) **EMBOLIZATION AGENT OR SCLEROSING AGENT FOR USE IN THE TREATMENT OF ERECTILE DYSFUNCTION**
EMBOLISATIONSMITTEL ODER SKLEROSIERUNGSMITTEL ZUR BEHANDLUNG VON EREKTILER DYSFUNKTION
AGENT D'EMBOLISATION OU AGENT SCLÉROSANT POUR LE TRAITEMENT DE LA DYSFONCTION ÉRECTILE

(30) Priority: 27.11.2023 EP 23212348
(43) Date of publication of application: 05.11.2025
(73) Proprietor: Garrido Pareja, Fermin Francisco, 18006 Granada (ES); Fernandez-Figares Suso, Maria Cristina, 18006 Granada (ES)
(72) Inventor: GARRIDO PAREJA, Fermin Francisco, 18006 Granada (ES)
(74) Representative: Grünecker Patent- und Rechtsanwälte PartG mbB
(86) International application number: PCT/EP2024/083815
(87) International publication number: WO 2025/114388

(56) References cited:
- WO-A2-2006/035319
- DUFFY DAVID M.: "Sclerosants A Comparative Review", DERMATOLOGIC SURGERY, vol. 36, no. Sup 2, 1 June 2010 (2010-06-01), Malden, USA, pages 1010 - 1025, XP093249050, ISSN: 1076-0512, Retrieved from the Internet <URL:https://onlinelibrary.wiley.com/doi/abs/10.1111%2Fj.1524-4725.2009.01469.x> DOI: 10.1111/j.1524-4725.2009.01469.x
- RALF HERWIG: "ERECTILE DYSFUNCTION AND CAVERNOUS VENO-OCCLUSIVE DISEASE", vol. 15, no. 2, 11 April 2019 (2019-04-11), AMSTERDAM, NL, pages 12, XP093156341, ISSN: 1875-6867, Retrieved from the Internet <URL:https://oss.jomh.org/files/article/20201210-56/pdf/67-Article%20Text-937-3-10-20190411.pdf> DOI: 10.22374/jomh.v15i2.67
- R HERWIG: "Erectile dysfunction and caverno-venous leak disease", vol. 4, no. 1, 15 January 2018 (2018-01-15), pages 1 - 5, XP093156415, ISSN: 2059-268X, Retrieved from the Internet <URL:https://www.oatext.com/pdf/JTS-4-205.pdf> DOI: 10.15761/JTS.1000205
- NAKATA ET AL: "Embolotherapy for Venous Impotence: Use of Ethanol", JOURNAL OF VASCULAR AND INTERVENTIONAL RADIOLOGY, ELSEVIER, AMSTERDAM, NL, vol. 11, no. 8, 1 September 2000 (2000-09-01), pages 1053 - 1057, XP022040866, ISSN: 1051-0443, DOI: 10.1016/S1051-0443(07)61338-4
- "Thursday, 2 November AM", BJU INTERNATIONAL, BLACKWELL SCIENCE, HOBOKEN, USA, vol. 86, 17 December 2008 (2008-12-17), pages 218 - 258, XP072216073, ISSN: 1464-4096, DOI: 10.1046/J.1464-410X.2000.0860S3218.X

## Description

### TECHNICAL FIELD OF THE INVENTION

The present invention relates to novel methods for treating erectile dysfunction by injecting an embolization agent or a sclerosing agent into the corpora cavernosa or corpus spongiosum of the penis of a patient. The present invention further relates to novel methods for treating erectile dysfunction by injecting an embolization agent or a sclerosing agent into one or more veins of the penis, wherein the embolization agent or sclerosing agent penetrates the corpora cavernosa or corpus spongiosum.

### BACKGROUND ART

Erectile dysfunction presents a clinical treatment challenge and no universally advocated treatment exists. Treatment attempts include both surgical and non-surgical approaches. Kaba et al. 2020 (Journal of Clinical Urology, Vol. 13(1), pp.33-39) describe that congenital venous leak or veno-occlusive dysfunction is an important cause of vasculogenic erectile dysfunction, posing a significant challenge to urologists.

Techniques for embolization of the draining veins and surgical management options based on resection or closing the incompetent veins have been described for the treatment of this pathology. During catheter embolization, a liquid embolization agent is applied using a catheter to occlude the deep dorsal vein close to the base of the penis. Until today, erectile dysfunction including erectile dysfunction of veno-occlusive origin, has been difficult to treat and penile prosthesis is often considered as the only solution.

One common venous leak treatment is medication, such as phosphodiesterase type 5 inhibitors, such as Viagra, which work by widening blood vessels, allowing blood to flow in faster than it leaks out. However, medication is merely temporary, may not be effective for everyone (e.g., men with low blood pressure or men who are on nitrate medications), and may have side effects for some individuals.

There is thus a need for novel methods of efficacious treatment of erectile dysfunction. The present application addresses this need with the novel methods for treating erectile dysfunction provided herein.

WO2006/035319A2 concerns a treatment of erectile dysfunction with chimeric coiled-coil molecules such as COMP-Angl, which are embolization agents. This document does not disclose an embolization agent or a sclerosing agent.

Herwig et a. JOURNAL OF MEN and APOS;S HEALTH,vol. 15, no. 2 11 April 2019 (2019-04-11), page 12, and JOURNAL OF TRANSLATIONAL SCIENCE,vol. 4, no. 1 15 January 2018 (2018-01-15), pages 1-5, respectively, are review articles on the treatment of veno-occlusive/vasculogenic erectile dysfunction via injection of polidocanol as sclerosing agent into deep dorsal vein of the penis.

Nakata et al., JOURNAL OF VASCULAR AND INTERVENTIONAL RADIOLOGY, ELSEVIER, AMSTERDAM, NL, vol. 11, no. 8, 1 September 2000 (2000-09-01), pages 1053-1057 and Da Silva et al., BJU INTERNATIONAL, BLACKWELL SCIENCE, HOBOKEN, USA, vol. 86, 17 December 2008 (2008-12-17), pages 218-258, respectively report on the use on ethanol as embolization agent for the treatment of erectile dysfunction. Both documents do not disclose a direct injection of the active agent into the corpus cavernosum or the corpus spongiosum.

### SUMMARY OF THE INVENTION

The present invention relates to novel methods for treating erectile dysfunction by injecting an embolization agent or a sclerosing agent into the corpora cavernosa or corpus spongiosum of the penis of a patient. The present invention further relates to novel methods for treating erectile dysfunction by injecting an embolization agent or a sclerosing agent into one or more veins of the penis, wherein the embolization agent or sclerosing agent penetrates the corpora cavernosa or corpus spongiosum.

The present invention is the first to describe the treatment of erectile dysfunction, in particular veno-occlusive erectile dysfunction, by targeting the corpora cavernosa or corpus spongiosum. Surprisingly, targeting the corpora cavernosa or corpus spongiosum with an embolization agent or a sclerosing agent has been found to be suitable for an efficacious treatment of erectile dysfunction, in particular veno-occlusive erectile dysfunction. The invention is based on a direct administration of an embolization agent or a sclerosing agent into the corpora cavernosa or corpus spongiosum, or the administration of an embolization agent or a sclerosing agent into one or more veins of the penis, in particular draining veins of the penis, respectively. It has surprisingly been found that the embolization agent or sclerosing agent can penetrate into the corpora cavernosa or corpus spongiosum from these draining veins.

Aspects and embodiments of the present invention include:
[1.] A method of treating erectile dysfunction comprising administering to a patient in need thereof a composition comprising an embolization agent or a sclerosing agent, wherein the composition is to be administered by injection into
   (a) one or more (draining) veins of the penis of the patient; and/or
   (b) the corpus cavernosum or the corpus spongiosum of the penis of the patient.
[2.] The method of [1(a)], wherein the one or more (draining) veins are selected from any one of the cavernosal vein(s), the deep dorsal vein, the bulbourethral vein(s), the para-arterial veins, and the circumflex veins.
[3.] The method of [1(a)] or [2], wherein the composition is forced to passage into the corpus cavernosum or the corpus spongiosum.
[4.] The method of any one of [1] to [3], wherein the one or more veins of the penis are compressed at a site proximal and/or distal from the site of injection.
[5.] The method of any one of [1] to [4], wherein the composition is to be administered by direct puncture into the one or more veins of the penis; or into the corpus cavernosum or the corpus spongiosum, respectively.
[6.] The method of any one of [1] to [5], wherein the composition is injected into:
   (i) the sinusoid cavity of the corpus cavernosum; and/or
   (ii) the sinusoid cavity of the corpus spongiosum.
[7.] The method of any one of [1] to [6], wherein the erectile dysfunction is veno-occlusive erectile dysfunction.
[8.] The method of [7], wherein the venous leakage associated with the veno-occlusive erectile dysfunction is determined by cavernosography, in particular dynamic infusion cavernosography (DIC), or ultrasound, in particular doppler ultrasound.
[9.] The method of any one of [1] to [8], comprising the occlusion of venous leaks upon administration of the composition comprising the embolization agent or the sclerosing agent.
[10.] The method of any one of [1] to [9], wherein the composition comprising the embolization agent is a liquid composition, preferably a liquid embolization agent.
[11.] The method of any one of [1] to [10], wherein the composition comprising the sclerosing agent is a liquid composition, a foam, or a microfoam, preferably a liquid sclerosing agent, a sclerosing foam, or a sclerosing microfoam.
[12.] The method of [11], wherein the sclerosing agent is polidocanol.
[13.] The method of any one of [1] to [12], further comprising imaging of the composition comprising the embolization agent or the sclerosing agent by ultrasound, or X-ray, or (CT- )cavernosography, preferably by ultrasound.
[14.] The method of any one of [1] to [13], wherein the penis of the patient is in non-erect state, or is in erect state induced by (injection of) a drug (that stimulates erection).

This summary does not necessarily describe all features of the present invention. Further aspects and embodiments of the present invention become apparent from a review of the ensuing detailed description.

### BRIEF DESCRIPTION OF THE DRAWINGS

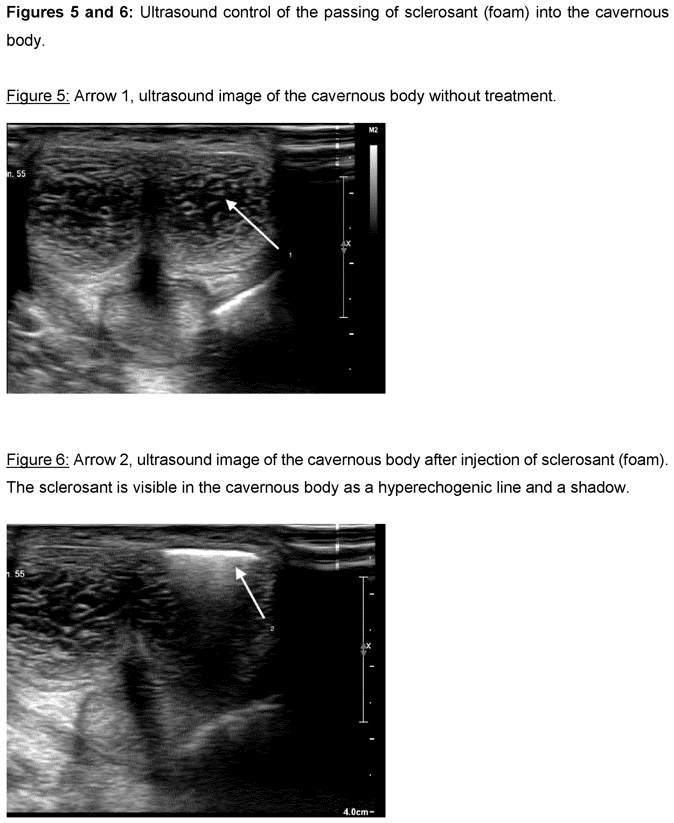

**Figure 1****:** is a cavernosography image showing a leak of the cavernous bodies to the pudendal vein (arrow 1). Direct puncture of the cavernous body near the leak (arrow 2) and injection of contrast material shows the drainage vein.
**Figures 2 and 3****:** show injection of sclerosant (foam) inside the cavernous body and its distribution, demonstrating how the contrast material disappears because the sclerosant (foam) is displacing the contrast material.
   Arrow 1 (Figure 2): Start of injection.
   Arrow 2 (Figure 3) The contrast material around the needle disappears due to the foam displacing it.
   Arrow 3 (Figure 3) No contrast material is seen in the periprostatic vein, thereby demonstrating the treatment.
**Figure 4****:** shows injection of contrast material into the cavernous body after treatment. The vein is spasmodic (arrow 1), and the contrast material can be seen in the cavernous body (arrow 2), confirming the treatment effect.
**Figures 5 and 6****:** show ultrasound control of the passing of sclerosant (foam) into the cavernous body.
   Arrow 1 (Figure 5) shows ultrasound image of the cavernous body without treatment.
   Arrow 2 (Figure 6) shows ultrasound image of the cavernous body after injection of sclerosant (foam). The sclerosant is visible in the cavernous body as a hyperechogenic line and a shadow. It has also been demonstrated that the sclerosant can penetrate the cavernous body from the deep dorsal vein. The same effect (i.e., penetration into the cavernous body) has been obtained with injection of sclerosant (foam) into the cavernosal vein(s), the bulbourethral vein(s), the para-arterial vein(s), and the circumflex vein(s).

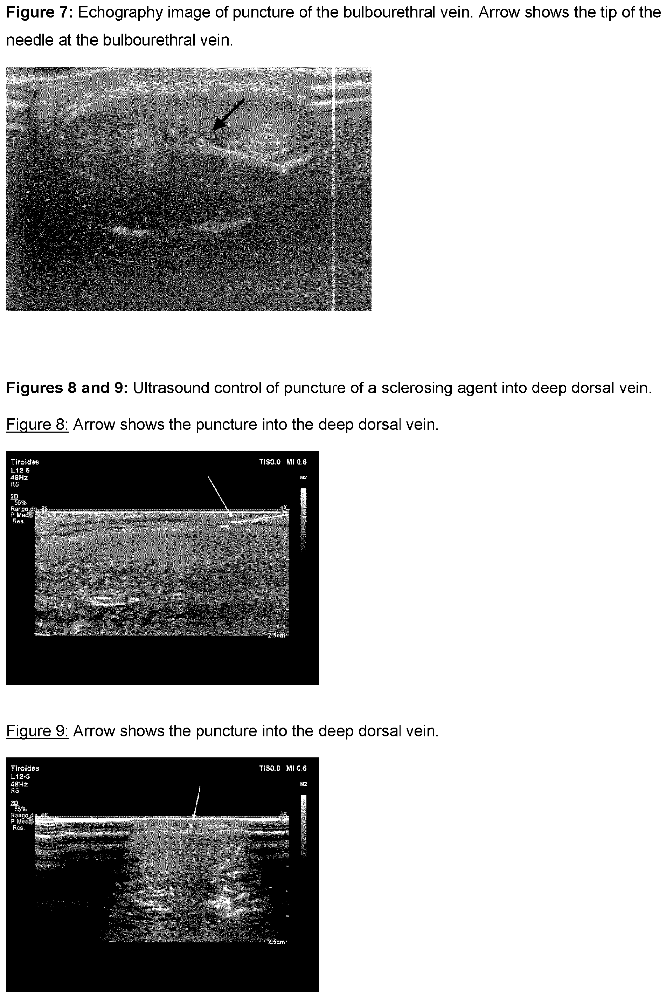

**Figure 7****:** shows an echography image of injection (puncture) of the bulbourethral vein. Arrow shows the tip of the needle at the bulbourethral vein.
**Figures 8 and 9****:** show ultrasound control of puncture of a sclerosing agent into the deep dorsal vein. In each of Figures 8 and 9: Arrow shows the puncture into the deep dorsal vein.

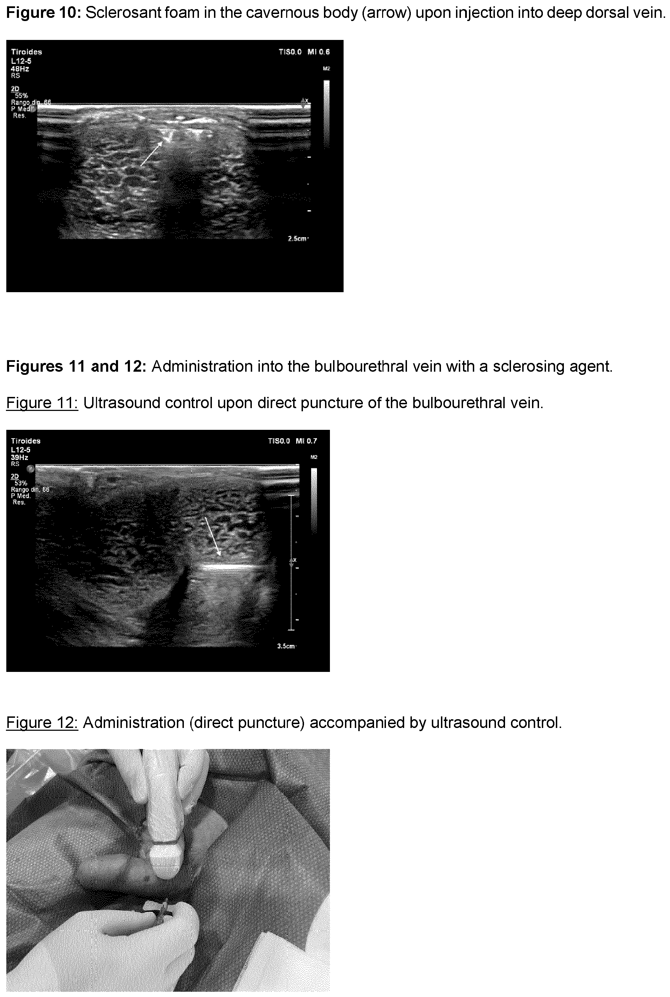

**Figure 10****:** shows sclerosant foam in the cavernous body (arrow) upon injection into the deep dorsal vein.
**Figures 11 and 12****:** show administration into the bulbourethral vein with a sclerosing agent. Figure 11: Ultrasound control of direct puncture of the bulbourethral vein. Figure 12: Administration (direct puncture) accompanied by ultrasound control.

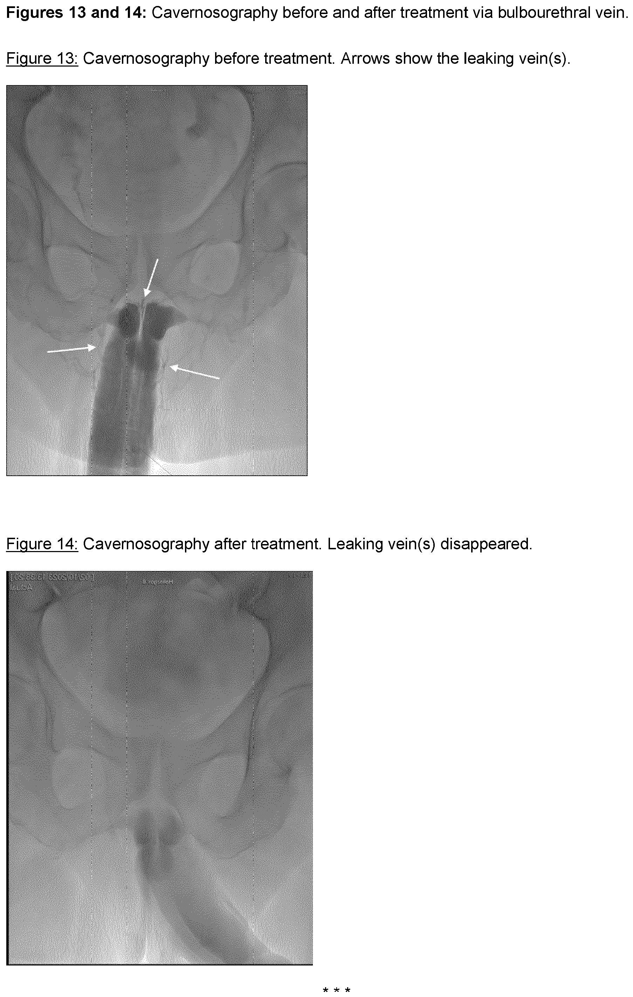

**Figures 13 and 14****:** show cavernosography before and after treatment via bulbourethral vein(s). Figure 13: Cavernosography before treatment. Arrows show the leaking vein(s). Figure 14: Cavernosography after treatment. Leaking vein(s) disappeared.

### DEFINITIONS

As described herein, the "corpus cavernosum" means the two chambers (sponge-like regions) of the penis that fill with blood to create an erection. As further described herein, the terms "corpus cavernosum" and "corpora cavernosa" may be used interchangeably. In particular, in various embodiments of the present invention, reference to the singular term "corpus cavernosum" includes both chambers, i.e., the plural term "corpora cavernosa". Accordingly, in various embodiments of the present invention, administration of an embolization agent or a sclerosing agent as described herein includes administration into both chambers, i.e., into the "corpora cavernosa". The same considerations apply with regard to the administration of an embolization agent or a sclerosing agent according to the invention into the sinusoid cavity of the corpus cavernosum. Accordingly, in various embodiments of the present invention, administration of an embolization agent or a sclerosing agent into the sinusoid cavity of the corpus cavernosum as described herein includes administration into the sinusoid cavity of both chambers, i.e., into the sinusoid cavity of the "corpora cavernosa".

Further, as described herein, the terms "corpus spongiosum" and "corpus cavernosum urethrae" may be used interchangeably.

The terms "sinusoid" and "sinusoid cavity", or "sinusoids" and "sinusoid cavities", respectively, may be used interchangeably herein.

As described herein, the terms "embolization agent" and "embolic agent" may be used interchangeably. As further described herein, the terms "embolization agent" and "embolization substance" may be used interchangeably.

As still further described herein, the terms "sclerosing agent" and "sclerosing substance" may be used interchangeably. As further described herein, a "sclerosing agent" or "sclerosing substance" may be considered as "sclerosant".

As used herein, the terms "composition comprising the embolization agent or sclerosing agent" and "embolization agent or sclerosing agent" may be used interchangeably herein. In particular, aspects and embodiments described herein that are referring to (the administration of) an "embolization agent or sclerosing agent" are considered to encompass the same aspects and embodiments where the "embolization agent or sclerosing agent" form part of a "composition comprising the embolization agent or sclerosing agent". The terms "(wherein the composition) is to be delivered" and "(wherein the composition) is to be administered" may be used interchangeably. Accordingly, the terms "(wherein the composition) is to be delivered by injection" and "(wherein the composition) is to be administered by injection" may be used interchangeably herein. In various embodiments, the terms "(wherein the composition) is to be administered by injection" and "(wherein the composition) is to be injected" may be used interchangeably.

### DETAILED DESCRIPTION

The present invention relates to novel methods for treating erectile dysfunction by injecting an embolization agent or a sclerosing agent directly into the corpora cavernosa or corpus spongiosum of the penis of a patient, or into one or more (draining) veins of the penis, wherein the embolization agent or sclerosing agent penetrates the corpora cavernosa or corpus spongiosum.

It has surprisingly been found that the claimed treatment is suitable to compensate for the structural lesions that cause impotence of veno-occlusive origin and affect the closure mechanism of the venules draining the sinusoids of the corpora cavernosa (and the corpus spongiosum). In particular, it has surprisingly been found that injecting an embolization agent or a sclerosing agent directly into the corpora cavernosa or the corpus spongiosum of the penis of a patient is not associated with severe side effects. For example, no thrombotic events have been observed when treatment of erectile dysfunction was performed by injecting an embolization agent or a sclerosing agent directly into the corpora cavernosa or corpus spongiosum of the penis of a patient.

It has furthermore surprisingly been found that upon injection into one or more draining veins of the penis, an embolization agent or a sclerosing agent penetrates the corpora cavernosa or corpus spongiosum, which in turn has been found to be suitable for treating erectile dysfunction. In particular, penetration of an embolization agent or a sclerosing agent into the corpora cavernosa or corpus spongiosum has been found not to be associated with severe side effects, for example, no thrombotic events have been observed, as explained herein above.

The present invention is the first to describe the treatment of erectile dysfunction, in particular veno-occlusive erectile dysfunction, by targeting the corpora cavernosa or corpus spongiosum. Surprisingly, targeting the corpora cavernosa or corpus spongiosum with an embolization agent or a sclerosing agent has been found to be suitable for an efficacious treatment of erectile dysfunction, in particular veno-occlusive erectile dysfunction. The invention is based on a direct administration of an embolization agent or a sclerosing agent into the corpora cavernosa or corpus spongiosum, or the administration of an embolization agent or a sclerosing agent into one or more draining veins of the penis. It has surprisingly been found that the embolization agent or sclerosing agent can penetrate into the corpora cavernosa or corpus spongiosum from these draining veins and thereby achieve a treatment effect for erectile dysfunction.

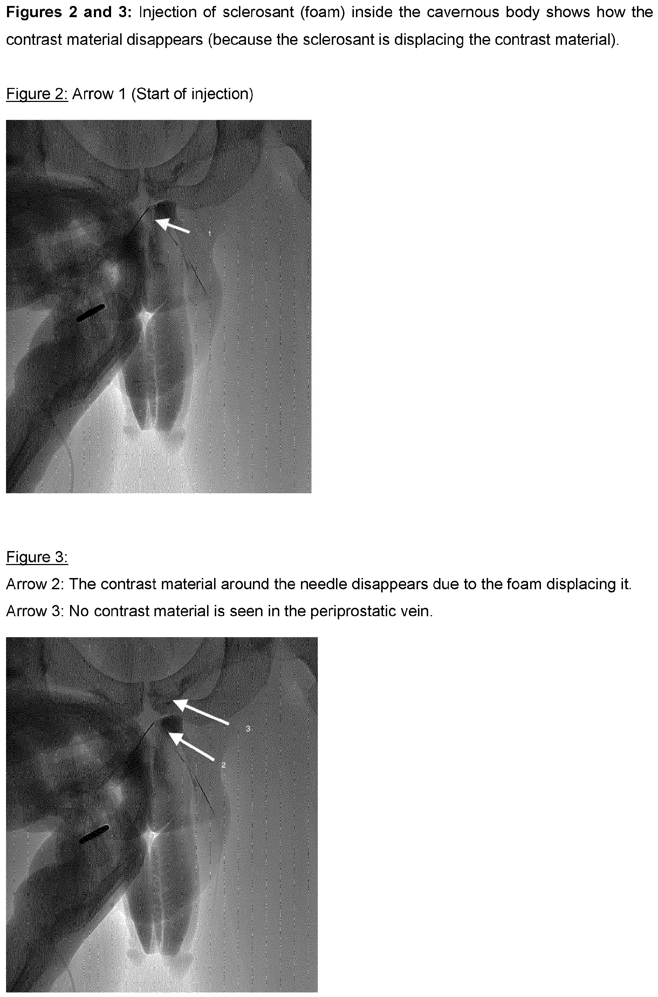

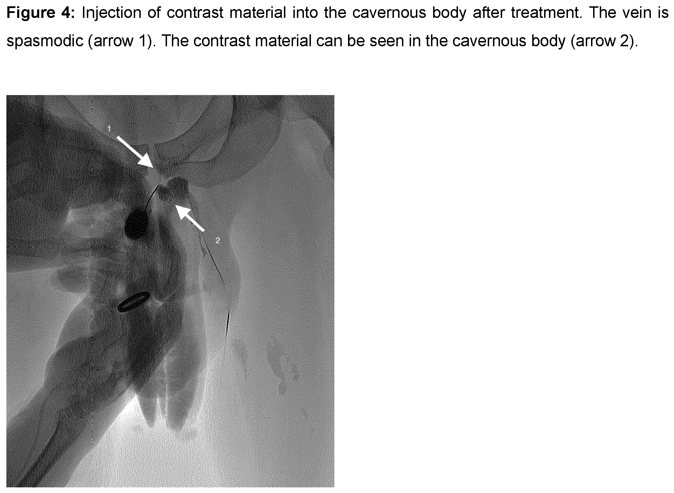

Accordingly, the claimed treatment is in particular suitable for treating erectile dysfunction, in particular veno-occlusive erectile dysfunction, by directly treating the corpora cavernosa, or by targeting the corpora cavernosa via one or more draining veins of the penis. Figures 2 and 3 show injection of sclerosant (foam) inside the cavernous body and its distribution. No contrast material is seen in the periprostatic vein (arrow 3 in Fig. 3), thereby demonstrating the treatment. Figure 4 shows injection of contrast material into the cavernous body after treatment. The vein is spasmodic (arrow 1 in Fig. 4), and the contrast material can be seen in the cavernous body (arrow 2), confirming the treatment effect. Figures 5 and 6 show ultrasound control of sclerosant foam in the cavernous body. Arrow 1 (Figure 5) shows an ultrasound image of the cavernous body without treatment. Arrow 2 (Figure 6) shows an ultrasound image of the cavernous body after injection of sclerosant. The sclerosant is visible in the cavernous body as a hyperechogenic line and a shadow.

It has also been demonstrated that the sclerosant can penetrate the cavernous body from the deep dorsal vein. Figures 8 and 9 show ultrasound control of puncture of a sclerosing agent into the deep dorsal vein. Figure 10 shows sclerosant foam in the cavernous body (arrow) upon injection into the deep dorsal vein.

The same effect of penetration into the cavernous body has been obtained with injection of sclerosant into the cavernosal vein(s), the bulbourethral veins, the para-arterial veins, and the circumflex veins. Those draining veins have not been described before as veins that can be used as entry veins for injection of an embolization agent or a sclerosing agent. Figure 7 shows an echography image of injection (puncture) of the bulbourethral vein (the arrow shows the tip of the needle at the bulbourethral vein). Figures 11 and 12 show administration into the bulbourethral vein with a sclerosing agent. Figure 11 shows an ultrasound image of a direct puncture of the bulbourethral vein. Figure 12 shows the administration (direct puncture) accompanied by ultrasound control.

As described herein, an embolization agent or a sclerosing agent that penetrates the corpora cavernosa or corpus spongiosum preferably means penetration of the sinusoids of the corpora cavernosa or of the sinusoids of the corpus spongiosum, respectively.

Accordingly, the claimed treatment is in particular suitable for treating erectile dysfunction, in particular veno-occlusive erectile dysfunction, by directly treating the sinusoids of the corpora cavernosa and/or the sinusoids of the corpus spongiosum, or by targeting the sinusoids of the corpora cavernosa, and/or the sinusoids of the corpus spongiosum, via one or more draining veins of the penis.

In various embodiments of the present invention, the embolization agent or sclerosing agent is administered into one or more draining veins when the penis is not in erect (rigid) state, or is not in complete erect (rigid) state. This facilitates the penetration of the embolization agent or sclerosing agent into the corpora cavernosa or corpus spongiosum when administered into a draining vein of the penis. In particular, in case of using a sclerosing agent, spasm of the one or more draining veins is (immediately) observed after administration/injection and facilitates the sclerosant to passage to the corpora cavernosa or corpus spongiosum. In case of using an embolization agent, in particular a liquid embolization agent, the agent acts as a plug and thereby facilitates the liquid embolization agent to passage to the corpora cavernosa or corpus spongiosum. More specifically, a (liquid) embolization agent may be injected to form a plug (at the point of leakage, or close to the point of leakage), and once a plug has formed (visible/detectable by X-ray, cavernosography, in particular DIC, or ultrasound, in particular doppler ultrasound), a (liquid) embolization agent then injected can passage to the corpora cavernosa (or corpus spongiosum). The passage to the corpora cavernosa (or corpus spongiosum) may be further facilitated by applying pressure, as described elsewhere herein. The embolization agent or sclerosing agent may be administered at a concentration of ≥ 10 mg/mL. In various embodiments, the embolization agent or sclerosing agent is administered into one or more draining veins when the penis is not in erect (rigid) state, or is not in complete erect (rigid) state, and the one or more draining veins may be compressed at a site proximal and/or distal from the site of administration (injection). In such embodiments, the one or more draining veins may be compressed at least at a site proximal from the site of administration (injection).

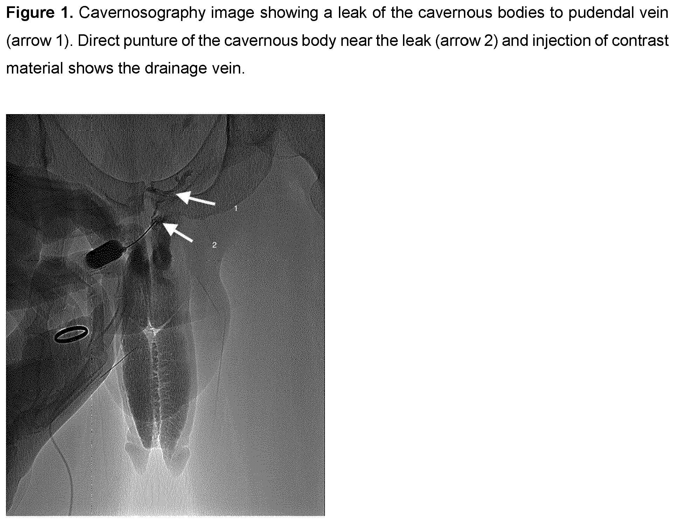

In various embodiments, the embolization agent or sclerosing agent is administered into one or more draining veins close to the (venous) leak of the respective vein(s). This also (or further) facilitates the penetration of the embolization agent or sclerosing agent into the corpora cavernosa or corpus spongiosum when administered into the one or more draining veins of the penis. In particular, in case of using a sclerosing agent, spasm of the one or more draining veins is observed (immediately) after administration/injection and (further) facilitates the sclerosant to passage to the corpora cavernosa or corpus spongiosum. In case of using an embolization agent, in particular a liquid embolization agent, the agent acts as a plug and thereby (further) facilitates the (liquid) embolization agent to passage to the corpora cavernosa or corpus spongiosum. The embolization agent or sclerosing agent may be administered into the one or more draining veins proximally and/or distally close to the (venous) leak of the respective vein(s). More specifically, a (liquid) embolization agent may be injected to form a plug (at the point of leakage, or close to the point of leakage), and once a plug has formed (visible/detectable by X-ray, cavernosography, in particular DIC, or ultrasound, in particular doppler ultrasound), a (liquid) embolization agent then injected can passage to the corpora cavernosa (or corpus spongiosum). The passage to the corpora cavernosa (or corpus spongiosum) may be further facilitated by applying pressure, as described elsewhere herein. The embolization agent or sclerosing agent may be administered at a concentration of ≥ 10 mg/mL. In various embodiments, the embolization agent or sclerosing agent is administered into the one or more draining veins (proximally and/or distally) close to the (venous) leak of the respective vein(s), and the one or more draining veins may be compressed at a site proximal and/or distal from the site of administration (injection). In such embodiments, the one or more draining veins may be compressed at least at a site proximal from the site of administration (injection). Figure 1 is a cavernosography image showing a leak of the cavernous bodies to the pudendal vein (arrow 1). Direct puncture of the cavernous body near the leak (arrow 2) and injection of contrast material shows the drainage vein.

It is preferred in the present invention that the embolization agent or sclerosing agent is administered into one or more draining veins close to the (venous) leak of the respective vein(s), when the penis is not in erect (rigid) state, or is not in complete erect (rigid) state. The embolization agent or sclerosing agent may be administered into the one or more draining veins proximally and/or distally close to the (venous) leak of the respective vein(s). The embolization agent or sclerosing agent may be administered at a concentration of ≥ 10 mg/mL. In various embodiments, the embolization agent or sclerosing agent is administered into the one or more draining veins (proximally and/or distally) close to the (venous) leak of the respective vein(s), when the penis is not in erect (rigid) state, or is not in complete erect (rigid) state, and the one or more draining veins may be compressed at a site proximal and/or distal from the site of administration (injection). In such embodiments, the one or more draining veins may be compressed at least at a site proximal from the site of administration (injection).

In various embodiments of the present invention, the embolization agent or sclerosing agent is administered into one or more draining veins close to the (venous) leak of the respective vein(s), when the penis is in (complete) erect (rigid) state. In this case, it is preferred that the one or more draining veins are compressed at a site proximal and/or distal from the site of administration (injection). In particular, in such case, it is preferred that the one or more draining veins are compressed at least at a site proximal from the site of administration (injection). In this case, it may further be preferred that the embolization agent or sclerosing agent is administered into the one or more draining veins proximally and/or distally close to the (venous) leak of the respective vein(s). The embolization agent or sclerosing agent may be administered at a concentration of ≥ 10 mg/mL.

Hence, when administered into one or more (draining) veins of the penis, the (composition comprising the) embolization agent or sclerosing agent is forced to passage into the corpora cavernosa or corpus spongiosum. The term "forced to passage into the corpora cavernosa or corpus spongiosum" in particular means that the one or more draining veins are compressed at a site proximal and/or distal from the site of administration (injection) to force the embolization agent or sclerosing agent to passage into the corpora cavernosa or corpus spongiosum. On the other hand, in various aspects or embodiments, the effect of the embolization agent or sclerosing agent can be considered to provide for (or result in) the embolization agent or sclerosing agent being "forced to passage into the corpora cavernosa or corpus spongiosum". The effect of the embolization agent or sclerosing agent comprises, or is based on, the occlusion of venous leaks upon administration of (the composition comprising) the embolization agent or the sclerosing agent. In particular, in case of a sclerosing agent, the effect of the sclerosing agent comprises, or is based on, spasm of the one or more draining veins that can be observed (immediately) after administration/injection. In case of an embolization agent, the effect comprises, or is based on, the embolization agent acting as a plug in the one or more draining veins that can be observed after administration/injection. In various embodiments, the occlusion of venous leaks upon administration of (the composition comprising) the sclerosing agent means occlusion of venous leaks by spasm and endothelial lesions of the one or more draining veins. In various embodiments, the occlusion of venous leaks upon administration of (the composition comprising) the embolization agent means occlusion of venous leaks by the embolization agent acting as a plug in the one or more draining veins. In preferred embodiments, the occlusion of venous leaks is induced and/or observed close to the (venous) leaks of the respective vein(s), more specifically proximally and/or distally close to the (venous) leaks of the respective vein(s). In other preferred embodiments, the spasm respectively plug is induced and/or observed close to the (venous) leak(s) of the respective vein(s), more specifically proximally and/or distally close to the (venous) leak(s) of the respective vein(s). The embolization agent or sclerosing agent may be administered at a concentration of ≥ 10 mg/mL.

The effect of the embolization agent or sclerosing agent described above, in particular the spasm respectively plug, can be imaged or determined by X-ray, cavernosography, (CT- )cavernosography, dynamic infusion cavernosography (DIC), or ultrasound, in particular doppler ultrasound. In case of an embolization agent, in particular a liquid embolization agent, it may be preferred to use X-ray, cavernosography, (CT-)cavernosography, dynamic infusion cavernosography (DIC) In case of a sclerosing agent, it may be preferred to use ultrasound, in particular doppler ultrasound.

It is preferred in the present invention that the embolization agent or sclerosing agent is administered/injected by direct puncture into the corpus cavernosum or the corpus spongiosum of the penis. In case of administration into one or more draining veins, it is also preferred that the embolization agent or sclerosing agent is administered by direct puncture into the one or more draining veins of the penis.

In particularly preferred embodiments, the embolization agent or sclerosing agent is administered/injected into the sinusoid cavity of the corpora cavernosa, and/or the sinusoid cavity of the corpus spongiosum. It is preferred that the embolization agent or sclerosing agent is administered/injected by direct puncture into the sinusoid cavity of the corpora cavernosa, and/or the sinusoid cavity of the corpus spongiosum.

In the present invention, reference is made equally to the corpora cavernosa and the corpus spongiosum. In various embodiments, the corpora cavernosa are preferred for administration over the corpus spongiosum. This preference may be considered for all aspects and embodiments disclosed herein. Accordingly, in various embodiments, administration into the sinusoid cavity/cavities of the corpora cavernosa is preferred over administration into the sinusoid cavity the corpus spongiosum.

In the present invention, reference is made equally to an embolization agent and a sclerosing agent. In various embodiments, the use of a sclerosing agent is preferred over the use of an embolization agent. In particular, in various embodiments, the use of a (liquid) sclerosing agent is preferred over the use of a liquid embolization agent. This preference may be considered for all aspects and embodiments disclosed herein. Accordingly, in various embodiments, administration of a sclerosing agent into the (sinusoid cavity of the) corpora cavernosa or corpus spongiosum may be preferred over the administration of an embolization agent. One may consider that the embolization is easier to control using X-rays. Further, one may consider that the sclerosing agent diffuses easier.

In various preferred embodiments of the present invention, the novel method is performed by administering an embolization agent, in particular a liquid embolization agent, to the corpora cavernosa, preferably to the sinusoid cavity/cavities of the corpora cavernosa.

In preferred embodiments of the present invention, injection into the corpora cavernosa or corpus spongiosum is accompanied by/comprises visual imaging of the corpora cavernosa or corpus spongiosum, respectively, to evaluate the dynamic movement of the cavernous space or the spongiosa space, respectively. In further preferred embodiments of the present invention, injection into the sinusoid cavity of the corpora cavernosa or corpus spongiosum is accompanied by/comprises visual imaging of the sinusoidal lumen to evaluate the dynamic movement of the cavernous space or the spongiosa space, respectively.

The effect of the embolization agent or sclerosing agent described above, in particular the occlusion of venous leaks upon administration of (the composition comprising) the embolization or sclerosing agent, can be imaged or determined by X-ray, cavernosography, (CT-)cavernosography, dynamic infusion cavernosography (DIC), or ultrasound, in particular doppler ultrasound. The occlusion of venous leaks imaged or determined encompasses imaging or determining spasm(s) and endothelial lesions (in case of a sclerosing agent) or plug(s) (in case of an embolization agent) by X-ray, cavernosography, (CT-)cavernosography, dynamic infusion cavernosography (DIC), or ultrasound, in particular doppler ultrasound. In case of an embolization agent, in particular a liquid embolization agent, it may be preferred to use X-ray, cavernosography, (CT-)cavernosography, dynamic infusion cavernosography (DIC). In case of a sclerosing agent, it may be preferred to use ultrasound, in particular doppler ultrasound. Figures 13 and 14 show cavernosography before and after treatment via bulbourethral vein(s). Figure 14 shows that the leaking vein(s) disappeared.

As described herein, treating or targeting the corpora cavernosa as described elsewhere herein includes both treating or targeting the two chambers (two corpora cavernosa), or only one of them (corpus cavernosum), whilst the preferred embodiment is treating or targeting both chambers (at the same time, or during the same treatment window). Thus, as used herein, reference to corpora cavernosa encompasses embodiments targeting or treating only one corpus cavernosum.

In preferred embodiments of the present invention, the one or more veins of the penis are selected from any one of the cavernosal vein(s), the deep dorsal vein, the bulbourethral vein(s), the para-arterial vein(s), and the circumflex vein(s). More preferably, the one or more veins of the penis are selected from the deep dorsal vein and the bulbourethral veins. In various embodiments, the one or more veins is/are the bulbourethral vein(s). Figure 7 shows injection (puncture) of the bulbourethral vein. Figures 8 and 9 show injection (puncture) of the deep dorsal vein. In various other preferred embodiments, the one or more veins are the para-arterial veins and/or the circumflex veins.

In certain embodiments of the invention, the one or more veins of the penis is the deep dorsal vein. Preferably, the embolization agent or sclerosing agent is injected into the deep dorsal vein by puncture, in particular by direct puncture. As described herein, direct puncture means direct puncture into the one or more veins of the penis described elsewhere herein.

Preferences regarding the (type of) draining veins: Administration into the cavernosal vein(s) may be preferred over administration into the deep dorsal vein. Administration into the bulbourethral veins, the para-arterial veins, and/or the circumflex veins, may be preferred over administration into the deep dorsal vein or the cavernosal vein(s). Administration into the bulbourethral veins is preferred over administration into the para-arterial veins, and/or the circumflex veins.

As further described herein, direct puncture is preferably performed using a needle, more specifically an injection needle, and preferably a sclerotherapy injection needle. In preferred embodiments, direct puncture is performed (with a needle as described elsewhere herein) under ultrasonography or under ultrasonography guidance.

As described herein, the injected dosis of sclerosing agent preferably does not exceed 2 mg/kg body weight per day. More specifically, the injected dosis of the sclerosing agent preferably does not exceed 2 mg/kg body weight per treatment. In preferred embodiments, the injected dosis of the sclerosing agent preferably does not exceed 2 mg/kg body weight per day/per treatment. The sclerosing agent preferably is polidocanol (lauromacrogol). The dosis of polidocanol preferably does not exceed 2 mg/kg body weight per treatment. In preferred embodiments, the injected dosis of the sclerosing agent preferably does not exceed 2 mg/kg body weight per day/per treatment. Preferred embolization agents are described elsewhere herein.

As further described herein, the dosis of an embolization agent, in particular a liquid embolization agent, injected into the corpora cavernosa or into the corpus spongiosum can be very small. The injected dosis of an embolization agent typically follows the manufacturer's recommendation. The volume of the (liquid) embolization agent administered according to the present invention does normally not exceed 1 mL per administration, and preferably is less than 1 mL per administration.

The volume of a foam or microfoam of the sclerosing agent (e.g., polidocanol) administered according to the present invention does normally not exceed 1.2 mL per administration, and preferably is less than 1.2 mL per administration.

As described herein, the treatment of erectile dysfunction according to the present invention excludes treatment by a catheter, or using a catheter. Accordingly, in various preferred embodiments, the treatment of erectile dysfunction according to the present invention excludes treatment by catheterization or using catheterization.

As discussed elsewhere herein, it has surprisingly been found that upon injection into one or more draining veins of the penis, an embolization agent or a sclerosing agent penetrates the corpora cavernosa or corpus spongiosum. In preferred embodiments, the embolization agent or sclerosing agent, or the composition comprising the embolization agent or sclerosing agent, is administered/injected into one (type of) draining vein of the penis. The preferences regarding the (type of) draining veins are described elsewhere herein.

As described elsewhere herein, the embolization agent or sclerosing agent, or the composition comprising the embolization agent or sclerosing agent, may be forced to passage into the corpus cavernosum or the corpus spongiosum by compression of the one or more draining veins. In this regard, the one or more veins may be compressed at a site proximal from the site of injection. In preferred embodiments, compression occurs on the deep dorsal vein, more preferably before the periprostatic plexus. In further (preferred) embodiments, the one or more veins may be compressed at a site distal from the site of injection. As described herein, the compression may be a manual compression of the one or more veins of the penis. The compression as described herein is not in itself to be considered as a method of treatment of erectile dysfunction. Accordingly, the compression as described herein may not be considered to constitute a method of treatment or surgery practiced on the human or animal body.

In the present invention, the treatment of erectile dysfunction preferably is treatment of veno-occlusive erectile dysfunction. As described herein, the terms veno-occlusive erectile dysfunction and veno-occlusive erectile disease may be used interchangeably herein. As further described herein, the terms veno-occlusive erectile dysfunction (or veno-occlusive erectile disease) and venogenic erectile dysfunction (or venogenic erectile disease) may be used interchangeably herein. Veno-occlusive erectile dysfunction is due to veno-occlusive dysfunction (or veno-occlusive insufficiency), also called "venous leak", characterized by an insufficient occlusion of venous outflow tracts. Additionally, or alternatively, venogenic erectile dysfunction may be considered to be due to insufficient penile blood retention during erection caused by venous leakage. In any case, in the present invention, veno-occlusive erectile dysfunction is characterized by (or associated with) venous leakage, in particular leakage of venous outflow tracts (of a patient's penis). More specifically, venous leakage means leakage of venous outflow tracts in the corpora cavernosa (or cavernosal tissue) of the penis.

As further described herein, venous leak may additionally or alternatively be considered as an inability to maintain an erection in the presence of sufficient arterial blood flow through the cavernosal arteries of the penis. As explained herein above, the defect is based on the excessive drainage of veins primarily in the cavernosal tissue of the penis, which undermines normal erectile function. The defect continues through the excessive drainage of veins around the outside wall of the corpora cavernosa. These veins include the deep dorsal vein, the bulbourethral veins, the para-arterial veins, and the circumflex veins. Accordingly, in various embodiments of the present invention, venous leakage means leakage of veins around the outside wall of the corpora cavernosa (or cavernosal tissue) of the penis. These veins include the deep dorsal vein, the bulbourethral veins, the para-arterial veins, and the circumflex veins. In various embodiments, the vein may be the pudendal vein. The pudendal vein may be treated by injection at the cavernous body.

As described herein, the novel treatment provided by the present invention provides for an occlusion of venous leaks upon administration of an embolization agent or sclerosing agent, or upon administration of a composition comprising the embolization agent or the sclerosing agent.

As further described herein, venous-occlusive dysfunction may be positively confirmed and demonstrated on CT cavernosography. In preferred embodiments, the treatment of the present invention may comprise a step of cavernosography, in particular CT cavernosography, prior to the administration of the (composition comprising the) embolization agent or sclerosing agent, to confirm the venous-occlusive dysfunction, and/or to visualize the one or more venous leak(s). In preferred embodiments of the present invention, the venous leakage associated with the veno-occlusive erectile dysfunction may be determined by cavernosography, in particular dynamic infusion cavernosography (DIC), or ultrasound, in particular doppler ultrasound. Preferred are cavernosography and accordingly also DIC.

As described herein, the one or more veins for administration (injection) of the embolization agent or sclerosing agent, can be any draining vein, which can be visualized with ultrasound or X-rays (using contrast material).

In preferred embodiments, the treatment of the present invention comprises an injection of the (composition comprising the) embolization agent or sclerosing agent at a site close to the one or more venous leak(s).

In preferred embodiments of the present invention, the composition comprising the embolization agent is a liquid composition, preferably a liquid embolization agent. In various preferred embodiments, the embolization agent is liquid and fills the vein, thereby obstructing it ("glue embolization"). Thus, the embolization agents do not necessarily damage the veins. In preferred embodiments, embolization agents are usually based on cyanoacrylate. The present invention encompasses cyanoacrylate liquid embolization agents, or cyanoacrylate glues, or cyanoacrylate liquid glues. The present invention in particular encompasses n-cyanoacrylate-based (liquid) embolization agents, including n-(hexyl) cyanoacrylate and n-(butyl) cyanoacrylate-based (liquid) embolization agents. The present invention further encompasses mixtures of n-cyanoacrylate and metacryloxysulpholane as (liquid) embolization agents, including mixtures of n-(hexyl) cyanoacrylate or n-(butyl) cyanoacrylate and metacryloxysulpholane as (liquid) embolization agents. The present invention further encompasses mixtures of n-cyanoacrylate and 2-octyl cyanoacrylate as (liquid) embolization agents, including mixtures of n-(hexyl) cyanoacrylate or n-(butyl) cyanoacrylate and 2-octyl cyanoacrylate as (liquid) embolization agents.

Liquid embolization agents typically solidify when they enter the vasculature. Accordingly, in case of using an embolization agent, in particular a liquid embolization agent, the agent may be considered to act as a plug, as described elsewhere herein.

Liquid embolization agents for use in the present invention may include, but are not limited to, ethanol, N-butyl cyanoacrylate (NBCA) glues, Lipiodol^{®} (an ethiodized oil) (Guerbet LLC) and ethiodized (poppy seed) oil-based embolization agents, and Onyx^{™} (Medtronic). Onyx^{™} is an embolic agent (fluid) made of EVOH (ethylene vinyl-alcohol copolymer), DMSO (dimethylsulfoxide) and TA (micronized tantalum powder). Accordingly, the present invention encompasses (liquid) embolization agents based on EVOH, preferably (liquid) embolization agents based on EVOH dissolved in DMSO, more preferably (liquid) embolization agents based on EVOH dissolved in DMSO with suspended micronized tantalum powder.

Liquid embolization agents disclosed herein encompass suitable combinations, e.g., a mixture of ethanol and ethiodized oil, which may be a 1:1 mixture of ethanol and ethiodized (poppy seed) oil. The terms "ethiodized oil" and "ethiodol" may be used interchangeably herein.

As described herein, the terms "embolization agent" and "embolic agent" may be used interchangeably herein.

Furthermore, in preferred embodiments of the present invention, the composition comprising the sclerosing agent is a liquid composition, a foam, or a microfoam, preferably a liquid sclerosing agent, a sclerosing foam, or a sclerosing microfoam. The sclerosing agent may be a non-ionic detergent, in particular a synthetic non-ionic detergent, such as sodium tetradecyl sulphate (STS). In preferred embodiments, the sclerosing agent may be a long-chain fatty alcohol, in particular a synthetic long-chain fatty alcohol. In more preferred embodiments, the sclerosing agent may be polidocanol (hydroxy-polyethoxy-dodecane; lauromacrogol). As described herein, a sclerosing agent may damage the inner wall of the vein(s). In the present invention, the use of a foam, in particular a microfoam, is preferred. Such (micro)foams may be prepared from any sclerosing substance with oxygen or a mixture of oxygen and carbon dioxide gas.

As described elsewhere herein, in preferred embodiments, the treatment of the present invention comprises a step of imaging of the (composition comprising the) embolization agent or the sclerosing agent by ultrasound, or X-ray, or (CT-) cavernosography, preferably by ultrasound.

In preferred embodiments, the treatment of the present invention comprises injection of the (composition comprising the) embolization agent or the sclerosing agent to a penis in non-erect (non-rigid) state, or in erect (rigid) state, wherein the erect (rigid) state may be induced by (injection of) a drug (that stimulates erection).

In various preferred embodiments of the present invention, patients receiving a treatment according to the invention do not need to take a PDE5 (Phosophodiesterase-5) inhibitor (in addition or parallel to the claimed treatment of erectile dysfunction). It has surprisingly been found that, with the treatment of the invention the erectile dysfunction is completely solved, and it will thus not be necessary for the patient to use any PDE5 inhibitor at all. In various embodiments, after the treatment according to the present invention, an erection occurs when complementing using a PDE5 inhibitor, i.e., both treatments complement each other. Accordingly, in various embodiments, the treatment of the present invention may comprise the administration of a PDE5 inhibitor to further support or to achieve an erection.

The present invention further encompasses:
[1.] A composition comprising an embolization agent or a sclerosing agent, for use in (a method of) treating erectile dysfunction, comprising administering the composition into a patient in need thereof, wherein the composition is to be administered (to the patient in need thereof) by injection into
   (a) one or more (draining) veins of the penis of the patient; and/or
   (b) the corpus cavernosum or the corpus spongiosum of the penis of the patient.
[2.] The composition for use in treating erectile dysfunction according to [1(a)], wherein the one or more (draining) veins are selected from any one of the cavernosal vein(s), the deep dorsal vein, the bulbourethral vein(s), the para-arterial veins, and the circumflex veins.
[3.] The composition for use in treating erectile dysfunction according to [1(a)] or [2], wherein the composition is forced to passage into the corpus cavernosum or the corpus spongiosum.
[4.] The composition for use in treating erectile dysfunction according to any one of [1] to [3], wherein the one or more veins of the penis are compressed at a site proximal from the site of injection.
[5.] The composition for use in treating erectile dysfunction according to any one of [1] to [4], wherein the one or more veins of the penis are compressed at a site distal from the site of injection.
[6.] The composition for use in treating erectile dysfunction according to any one of [1] to [5], wherein the composition is to be administered by direct puncture into the one or more veins; or into the corpus cavernosum or the corpus spongiosum, respectively.
[7.] The composition for use in treating erectile dysfunction according to any one of [1] to [6], wherein the composition is injected into:
   (i) the sinusoid cavity of the corpus cavernosum; and/or
   (ii) the sinusoid cavity of the corpus spongiosum.
[8.] The composition for use in treating erectile dysfunction according to any one of [1] to [7], wherein the erectile dysfunction is veno-occlusive erectile dysfunction.
[9.] The composition for use in treating erectile dysfunction according to [8], wherein the venous leakage associated with the veno-occlusive erectile dysfunction is determined by cavernosography, in particular dynamic infusion cavernosography (DIC), or ultrasound, in particular doppler ultrasound.
[10.] The composition for use in treating erectile dysfunction according to any one of [1] to [9], comprising the occlusion of venous leaks upon administration of the composition comprising the embolization agent or the sclerosing agent.
[11.] The composition for use in treating erectile dysfunction according to any one of [1] to [10], wherein the composition comprising the embolization agent is a liquid composition, preferably a liquid embolization agent.
[12.] The composition for use in treating erectile dysfunction according to any one of [1] to [10], wherein the composition comprising the sclerosing agent is a liquid composition, a foam, or a microfoam, preferably a liquid sclerosing agent, a sclerosing foam, or a sclerosing microfoam.
[13.] The composition for use in treating erectile dysfunction according to any one of [1] to [10] and [12], wherein the sclerosing agent is polidocanol.
[14.] The composition for use in treating erectile dysfunction according to any one of [1] to [13], further comprising imaging of the composition comprising the embolization agent or the sclerosing agent by ultrasound, or X-ray, or (CT-)cavernosography, preferably by ultrasound.
[15.] The composition for use in treating erectile dysfunction according to any one of [1] to [14], wherein the penis of the patient is in non-erect state, or is in erect state induced by (injection of) a drug (that stimulates erection).

Embodiments of the methods of the present invention are illustrated in the following examples. These examples are provided for illustrative purposes and are not considered limitations on the scope of the methods and uses of the invention.

### EXAMPLES

### Example 1: Cavernosography identifies veno-occlusive erectile dysfunction

Patients (male, human) with erectile dysfunction are subjected to cavernosography to identify venous leaks. Figure 1 is a cavernosography image and shows a leak of the cavernous bodies to pudendal vein (arrow 1). Direct puncture of the cavernous body near the leak (arrow 2) and injection of contrast material shows the drainage vein. The patients are suffering from veno-occlusive erectile dysfunction.

### Example 2: Proof of concept of treatment in the cavernous body

Patients (male, human) with erectile dysfunction are subjected to injection of a sclerosing agent (sclerosant, foam) inside the cavernous body. Prior to injection of the sclerosant, all patients received an injection of contrast material to verify the treatment effect with the subsequently injected sclerosant. Figures 2 and 3 show injection of sclerosant (foam) inside the cavernous body and its distribution, demonstrating how the contrast material disappears because the sclerosant (foam) is displacing the contrast material. Arrow 1 in Figure 2 shows the start of the injection of the sclerosant. Arrow 2 in Figure 3 shows that the contrast material around the needle disappears due to the foam displacing it. Arrow 3 in Figure 3 shows that no contrast material is present in the periprostatic vein, thereby demonstrating the treatment.

Figures 5 and 6 show ultrasound images proving the passing of sclerosant (foam) into the cavernous body. Arrow 1 (Figure 5) shows an ultrasound image of the cavernous body without treatment. Arrow 2 (Figure 6) shows an ultrasound image of the cavernous body after injection of sclerosant (foam). The sclerosant is visible in the cavernous body as a hyperechogenic line and a shadow.

Example 2 exemplifies the treatment of the invention according to which an embolization agent or a sclerosing agent is injected into the corpora cavernosa. For all patients treated, it is possible to get an erection after treatment. The erection is shown to occur normal in time, as is the hardness. The erection is shown to be maintained over time ("long lasting") due to the leaking vein(s) treated (no reappearance).

### Example 3: Patients with induced erection prior to treatment

In a separate trial having the same set up as in Example 2, an erection is induced in the patients (male, human) prior to treatment. For all patients treated, it is possible to maintain the erection after treatment. The erection is shown to occur normal in time, as is the hardness. The erection is shown to be maintained over time ("long lasting") due to the leaking vein(s) treated (no reappearance).

### Example 4: Injection into the bulbourethral veins, the para-arterial veins, and the circumflex veins

In a trial having the same set up as in Example 2, the sclerosant is injected into any of the bulbourethral veins, the para-arterial veins, and the circumflex veins. Figure 7 shows an echography image of injection (puncture) into the bulbourethral vein. The arrow shows the tip of the needle at the bulbourethral vein.

The same effect as demonstrated in Example 2 has been obtained with injection of the sclerosant (foam) into the bulbourethral vein(s), the para-arterial vein(s), and the circumflex vein(s). Figures 11 and 12 are images showing the administration of sclerosant foam into the bulbourethral vein. Figure 11 shows an ultrasound image of direct puncture of the bulbourethral vein. Figure 12 shows the administration (direct puncture) accompanied by ultrasound control. Figures 13 and 14 are cavernosography images before and after treatment via bulbourethral vein(s). In Figure 13, the arrows show the leaking veins. Figure 14 shows that the leaking veins disappeared after treatment. Figures 13 and 14 verify the passing of the scleorsant into the corpora cavernosa, i.e., verify that the treatment is correct.

Thus, Example 4 further confirms the treatment of the invention according to which an embolization agent or a sclerosing agent is injected into one or more draining veins of the penis of a patient. For all patients treated in Example 4, it is possible to get an erection after treatment. The erection is shown to occur normal in time, as is the hardness. The erection is shown to be maintained over time ("long lasting") due to the leaking vein(s) treated (no reappearance).

### Example 5: Injection into the deep dorsal vein and passing into the corpora cavernosa

In a trial having the same set up as in Example 2 or Example 4, the sclerosant is injected into the deep dorsal vein. Figures 8 and 9 show ultrasound images of puncture of a sclerosing agent into the deep dorsal vein. In each of Figures 8 and 9, the arrow shows the puncture into the deep dorsal vein. Figure 10 shows sclerosant foam in the cavernous body (arrow) upon injection into the deep dorsal vein, thereby showing the passing of the scleorsant into the corpora cavernosa.

Hence, the same effect as demonstrated in Example 4 has been obtained with injection of the sclerosant (foam) into the deep dorsal vein.

Thus, Example 5 further confirms the treatment of the invention according to which an embolization agent or a sclerosing agent is injected into one or more draining veins of the penis of a patient. For all patients treated in Example 5, it is possible to get an erection after treatment. The erection is long lasting.

### Example 6: Further verification of treatment

The treatment effect is further verified by injection of contrast material after treatment. Figure 4 shows injection of contrast material into the cavernous body after treatment. Arrow 1 in Figure 4 shows the spasmodic vein, and the contrast material can be seen in the cavernous body (arrow 2), confirming the treatment effect.

## Claims

1. A composition comprising an embolization agent or a sclerosing agent for use in a method of treating veno-occlusive erectile dysfunction, comprising administering the composition to a patient in need thereof, wherein the composition is to be administered by injection into the corpus cavernosum or the corpus spongiosum of the penis of the patient.

2. The composition for use according to claim 1, wherein the composition is to be administered by direct puncture into the corpus cavernosum or the corpus spongiosum.

3. The composition for use according to claim 1 or 2, wherein the composition is injected into:
(i) the sinusoid cavity of the corpus cavernosum; and/or
(ii) the sinusoid cavity of the corpus spongiosum.

4. The composition for use according to any one of claims 1 to 3, wherein the venous leakage associated with the veno-occlusive erectile dysfunction is determined by cavernosography, in particular dynamic infusion cavernosography (DIC), or ultrasound, in particular doppler ultrasound.

5. The composition for use according to any one of claims 1 to 4, comprising the occlusion of venous leaks upon administration of the composition comprising the embolization agent or the sclerosing agent.

6. The composition for use according to any one of claims 1 to 5, wherein the composition comprising the embolization agent is a liquid composition, preferably a liquid embolization agent.

7. The composition for use according to any one of claims 1 to 5, wherein the composition comprising the sclerosing agent is a liquid composition, a foam, or a microfoam, preferably a liquid sclerosing agent, a sclerosing foam, or a sclerosing microfoam.

8. The composition for use according to any one of claims 1 to 5 and 7, wherein the sclerosing agent is polidocanol.

9. The composition for use according to any one of claims 1 to 8, further comprising imaging of the composition comprising the embolization agent or the sclerosing agent by ultrasound, or X-ray, or (CT-)cavernosography, preferably by ultrasound.

10. The composition for use according to any one of claims 1 to 9, wherein the penis of the patient is in non-erect state, or is in erect state induced by (injection of) a drug (that stimulates erection).

## Patentansprüche

1. Zusammensetzung, umfassend ein Embolisationsmittel oder ein Sklerosierungsmittel, zur Verwendung bei einem Verfahren zur Behandlung einer venookklusiven erektilen Dysfunktion, umfassend die Verabreichung der Zusammensetzung an einen Patienten, der sie benötigt, wobei die Zusammensetzung durch Injektion in den Corpus cavernosum oder den Corpus spongiosum des Penis des Patienten zu verabreichen ist.

2. Die Zusammensetzung zur Verwendung gemäß Anspruch 1, wobei die Zusammensetzung durch direkte Punktion in den Corpus cavernosum oder den Corpus spongiosum zu verabreichen ist.

3. Die Zusammensetzung zur Verwendung gemäß Anspruch 1 oder 2, wobei die Zusammensetzung injiziert wird in:
(i) die Sinusoidhöhle des Corpus cavernosum; und/oder
(ii) die Sinusoidhöhle des Corpus spongiosum.

4. Die Zusammensetzung zur Verwendung gemäß einem der Ansprüche 1 bis 3, wobei die mit der venookklusiven erektilen Dysfunktion verbundene venöse Leckage durch Kavernosographie, insbesondere dynamische Infusionskavernosographie (DIC), oder Ultraschall, insbesondere Doppler-Ultraschall, bestimmt wird.

5. Die Zusammensetzung zur Verwendung gemäß einem der Ansprüche 1 bis 4, umfassend die Okklusion venöser Leckagen nach Verabreichung der Zusammensetzung umfassend das Embolisationsmittel oder das Sklerosierungsmittel.

6. Die Zusammensetzung zur Verwendung gemäß einem der Ansprüche 1 bis 5, wobei die Zusammensetzung, welche das Embolisationsmittel umfasst, eine flüssige Zusammensetzung, vorzugsweise ein flüssiges Embolisationsmittel, ist.

7. Die Zusammensetzung zur Verwendung gemäß einem der Ansprüche 1 bis 5, wobei die Zusammensetzung, welche das Sklerosierungsmittel umfasst, eine flüssige Zusammensetzung, ein Schaum oder ein Mikroschaum ist, vorzugsweise ein flüssiges Sklerosierungsmittel, ein Sklerosierungsschaum oder ein Sklerosierungsmikroschaum.

8. Die Zusammensetzung zur Verwendung gemäß einem der Ansprüche 1 bis 5 und 7, wobei das Sklerosierungsmittel Polidocanol ist.

9. Die Zusammensetzung zur Verwendung gemäß einem der Ansprüche 1 bis 8, weiterhin umfassend eine Bildgebung der Zusammensetzung, welche das Embolisationsmittel oder das Sklerosierungsmittel umfasst, mittels Ultraschall, Röntgenstrahlen oder (CT-)Kavernosographie, vorzugsweise mittels Ultraschall.

10. Die Zusammensetzung zur Verwendung gemäß einem der Ansprüche 1 bis 9, wobei sich der Penis des Patienten in einem nicht erigierten Zustand befindet oder sich in einem durch (die Injektion) ein(es) Medikament(s) (welches die Erektion stimuliert) induzierten erigierten Zustand befindet.

## Revendications

1. Composition comprenant un agent embolisant ou un agent sclérosant destiné à être utilisé dans un procédé de traitement de la dysfonction érectile veino-occlusive, comprenant l'administration de la composition à un patient qui en a besoin, dans laquelle la composition doit être administrée par injection dans le corps caverneux ou le corps spongieux du pénis du patient.

2. Composition destinée à être utilisée selon la revendication 1, dans laquelle la composition doit être administrée par ponction directe dans le corps caverneux ou le corps spongieux.

3. Composition destinée à être utilisée selon la revendication 1 ou 2, dans laquelle la composition est injectée dans :
(i) la cavité sinusoïdale du corps caverneux ; et/ou
(ii) la cavité sinusoïdale du corps spongieux.

4. Composition destinée à être utilisée selon l'une quelconque des revendications 1 à 3, dans laquelle la fuite veineuse associée à la dysfonction érectile veino-occlusive est déterminée par cavernosographie, en particulier par cavernosographie à perfusion dynamique (DIC), ou par échographie, en particulier par échographie Doppler.

5. Composition à utiliser selon l'une quelconque des revendications 1 à 4, comprenant l'occlusion des fuites veineuses lors de l'administration de la composition comprenant l'agent embolisant ou l'agent sclérosant.

6. Composition destinée à être utilisée selon l'une quelconque des revendications 1 à 5, dans laquelle la composition comprenant l'agent d'embolisation est une composition liquide, de préférence un agent d'embolisation liquide.

7. Composition destinée à être utilisée selon l'une quelconque des revendications 1 à 5, dans laquelle la composition comprenant l'agent sclérosant est une composition liquide, une mousse ou une micro-mousse, de préférence un agent sclérosant liquide, une mousse sclérosante ou une micro-mousse sclérosante.

8. Composition destinée à être utilisée selon l'une quelconque des revendications 1 à 5 et 7, dans laquelle l'agent sclérosant est le polidocanol.

9. Composition destinée à être utilisée selon l'une quelconque des revendications 1 à 8, comprenant en outre l'imagerie de la composition comprenant l'agent embolisant ou l'agent sclérosant par ultrasons, ou rayons X, ou cavernosographie (CT), de préférence par ultrasons.

10. Composition destinée à être utilisée selon l'une quelconque des revendications 1 à 9, dans laquelle le pénis du patient est à l'état non érigé, ou est à l'état érigé induit par (l'injection d'un) médicament (qui stimule l'érection).
